# EUROPÄISCHE PATENTSCHRIFT

(11) **EP 1 294 419 B1**
(45) Veröffentlichungstag und Bekanntmachung des Hinweises auf die Patenterteilung: **04.05.2005**
(21) Anmeldenummer: 01965018.3
(22) Anmeldetag: 21.06.2001
(51) Int. Cl.: A61M 5/32, A61B 17/34, A61B 19/00, G01R 33/28

(54) **VORRICHTUNG ZUR INJEKTION VON MEDIZINISCHEN PRÄPARATEN UNTER CT-/MRT KONTROLLE**
DEVICE FOR INJECTION OF MEDICAL PREPARATIONS WITH CT/MRI MONITORING
DISPOSITIF D'INJECTION DE PREPARATIONS MEDICINALES SOUS LA SURVEILLANCE D'UN TOMODENSITOMETRE/TOMOGRAPHE A RESONANCE MAGNETIQUE

(30) Priorität: 28.06.2000 DE 10030620
(43) Veröffentlichungstag der Anmeldung: 26.03.2003
(73) Patentinhaber: Forschungszentrum Karlsruhe GmbH, 76133 Karlsruhe (DE)
(72) Erfinder: GUMB, Lothar, 76676 Graben-Neudorf (DE); HANNULA, Henri, FI-20780 Kaarina (FI); KÖHN, Swen, 76149 Karlsruhe (DE); MELZER, Andreas, 45478 Mülheim/Ruhr (DE); SCHÄF, Aribert, 76646 Bruchsal (DE); PROKOTT, Georg, 76344 Eggestein-Leopoldshafen (DE)
(86) Internationale Anmeldenummer: PCT/EP2001/006996
(87) Internationale Veröffentlichungsnummer: WO 2002/000276

(56) Entgegenhaltungen:
- WO-A-98/23213
- DE-C- 19 855 293
- US-A- 5 469 847

## Beschreibung

Die vorliegende Erfindung betrifft eine Vorrichtung für eine Injektion von medizinischen Präparaten, insbesondere Medikamenten oder Kontrastmitteln, in einen Patienten-körper während einer Untersuchung im Computertomographen, CT, Magnetresonaztomographen, MRT, oder mit Endoskop gemäß des Oberbegriffs des Anspruch 1. Die vorliegende Erfindung umfasst dabei auch einen Einsatz der Vorrichtung in einem Magnetresonanztompgraphen, MRT.

Bei der interventionellen Radiologie orientiert und überwacht der Arzt seine operativen Eingriffe mit Hilfe von bildgebenden Verfahren, beispielsweise mit Hilfe der CT und MRT. Üblicherweise liegt der Patient dabei auf einer Patientenliege, während der behandelnde Arzt den Eingriff am Bildschirm überwacht. Bei tomographischen Verfahren, wie z.B. der CT- und MRT-Verfahren sind dies zweidimensionale Schnittbilder durch den Patientenkörper, welche im geringen Abstand zueinander aufgenommen werden und zu einem dreidimensionalen Abbild, dem sogenannten Tomogramm, des Patientenkörper zusammengesetzt werden. Die erstellten Tomogramme zeigen die inneren Organe, Gewebe- und Knochenstrukturen im Detail. Dabei können beliebige Schnittbilder generiert werden. Zudem lassen sich beim CT durch Kippen der Gantry oder programmgesteuert beim MRT individuelle Schnittdarstellungen generieren.

Anhand der tomographischen Daten legt der behandelnde Arzt seine Zugangsplanung des anschließend zu operierenden Organs fest und ermittelt im Falle einer bildgestützten und manipulatorgesteuerten Medikamentendosierung die Koordinaten und Dosierraten für den Einstich der Injektionsnadel und sendet diese an den Manipulator.

Aus [1] ist ein derartiges Medikamentendosierungssystem bekannt, bei dem von zwei 65 ml Einwegspritzen Medikamente oder Kontrastmittel im wesentlichen über einen Schlauch und eine Injektionsnadel in den Patientenkörper injiziert werden. Die Betätigung der Einwegspritzen erfolgt, wie die technischen Angaben in [1] vermuten lassen, wahrscheinlich elektromotorisch über Linearantriebe. Ferner wird ein naheliegender Einsatz des Medikamentendosiersystems im MRT oder CT in [1] unter tomographischer Kontrolle nicht erwähnt, obwohl die Baugröße für einen Einsatz im Kanal eines Tomographen durchaus geeignet erscheint.

Ein manipulatorgesteuertes Medikamentendosierungssystem mit Injektionsnadel, welches für den Einsatz innerhalb eines CT's oder MRT's geeignet ist, ist dagegen nicht bekannt.

Der Erfindung liegt die Aufgabe zugrunde, eine Vorrichtung für eine Injektion von medizinischen Präparaten gegenüber des zuletzt genannten Stands der Technik so zu modifizieren, dass diese als gesamte Einheit in einen Tomographen einschiebbar und in diesem bildgestützt einsetzbar ist.

Die Aufgabe wird durch die in Anspruch 1 beschriebene Vorrichtung gelöst. Die weiteren Ansprüche geben bevorzugte Ausgestaltungen der Vorrichtung an.

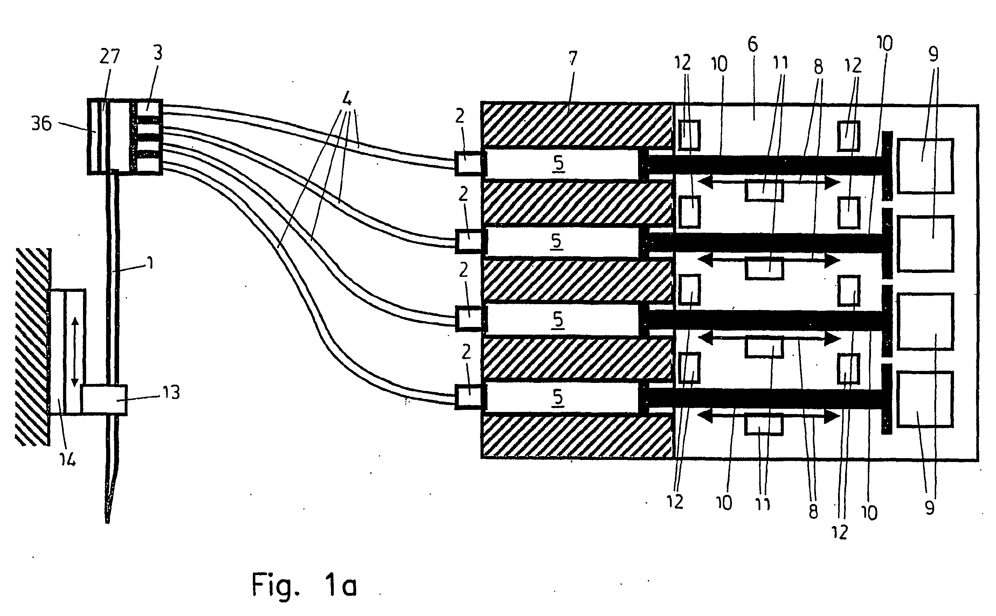

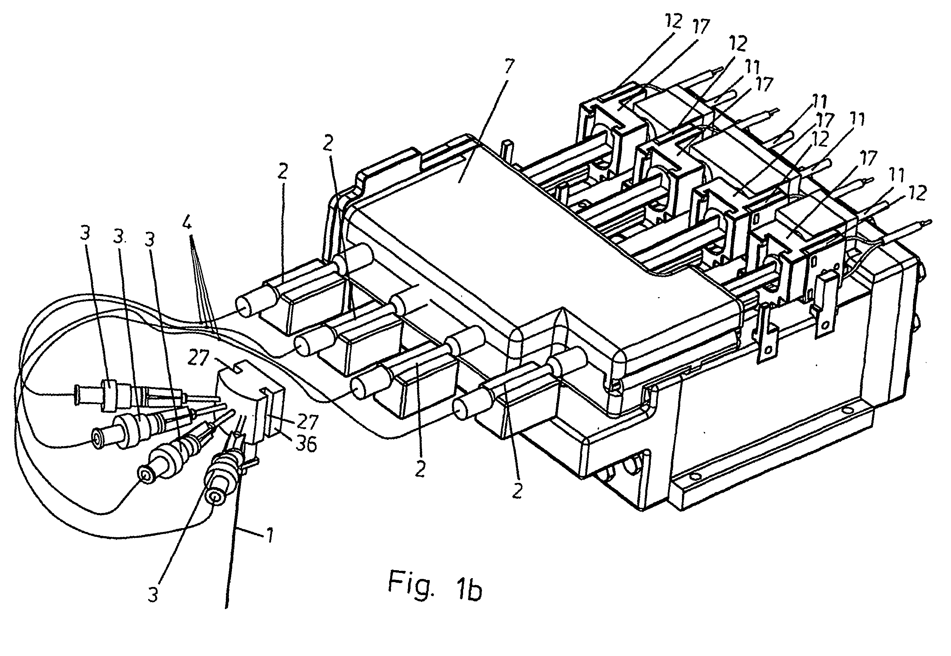

Die erfindungsgemäße Vorrichtung für eine Injektion von medizinischen Präparaten wird im folgenden anhand von Zeichnungen einer Ausführungsform mit vier Einwegspritzen erläutert. Es zeigen
Fig.1 a die Komponenten der Vorrichtung für eine Injektion von medizinischen Präparaten als Prinzipskizze,
Fig. 1 b die Vorrichtung gemäß Fig. 1 a in perspektivischer Darstellung, jedoch ohne den Nadelgreifer 13 und Verfahrvorrichtung 14,
Fig. 2 die Dosiervorrichtung 6 gemäß Fig. 1 b in perspektivischer Schnittdarstellung,
Fig. 3 a und b die Injektionsnadelvorschubvorrichtung ohne Injektionsnadel 1 als Schnittdarstellung mit unterschiedlichen Positionen des Nadelgreifers 13,
Fig. 4 a und b die Injektionsnadelvorschubvorrichtung als perspektivische Darstellung mit unterschiedlichen Positionen des Nadelgreifers 13,
Fig. 5 eine Schnittdarstellung der Nadelgreifer 13, sowie
Fig. 6 die bestimmungsgemäße Positionierung der Vorrichtung für eine Injektion von medizinischen Präparaten auf einem Träger oberhalb eines Patienten 35.

Die beschriebene Ausführungsform der Vorrichtung besteht, wie in den Figuren 1 a und 1 b dargestellt, aus einer Infusionsnadel 1, welche über eine Mischkammer 36, vier Rückschlagventile 3, vier Schläuche 4 und vier Drucksensoren 2 an vier Einwegspritzen 5 der Dosiervorrichtung 6 angeschlossen ist. Dabei sind die vier Einwegspritzen 5 in der Dosiervorrichtung 6 in ein Wechselmagazin 7 reversibel einlegbar und über je einen Antrieb 9, welcher jeden Kolben 10 jeder Einwegspritze 5 einzeln in beiden Richtungen 8 verschieben kann, betätigbar. Solange jedoch zwischen jeder Einwegspritze 5 und der Infusionsnadel 1 ein einen Rückfluss verhinderndes Rückschlagventil 3 geschaltet ist, ist eine Betätigung in beiden Richtungen ohne ein Umdrehen oder Austausch des Rückschlagventils 3 nicht möglich. Bei Betätigung einer Spritze wird der Injektionsfluss über die Zustellbewegung des Kolbens 10 über einen Wegsensor 11 sowie über eine Druckmessung am Drucksensor 2 kontinuierlich überwacht. Endschalter 12 dienen dabei der Begrenzung der Zustellbewegung der Kolben 10 in jede der beiden Richtungen 8.

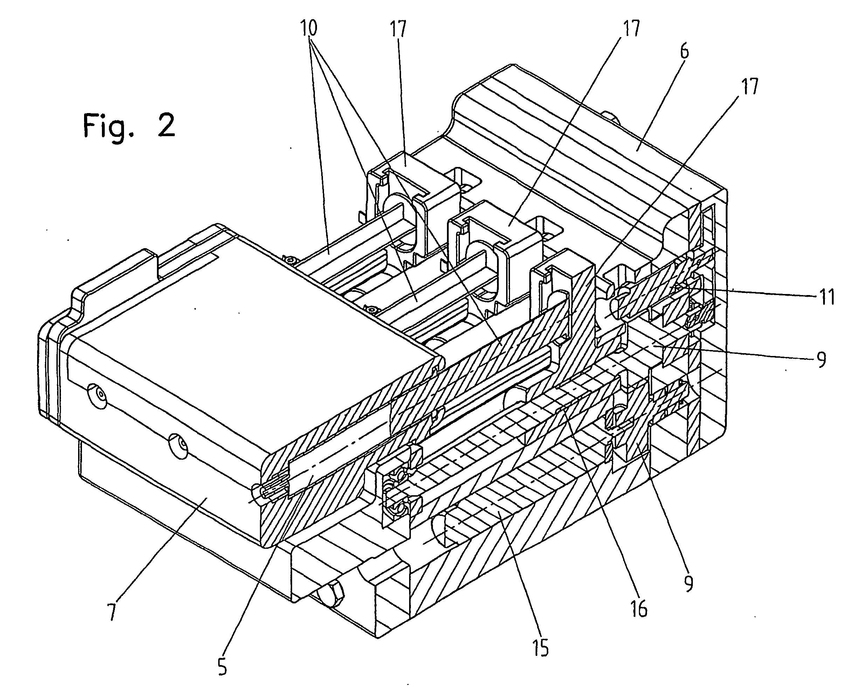

Im Gegensatz zu der in Fig. 1 a dargestellten Prinzipskizze ist in der perspektivischen Darstellung gemäß Fig. 1 b der Wegsensor 11 hinter dem Kolben 10 angeordnet, während der Antrieb 9 unterhalb der Kolben 10 positioniert ist und über Mitnehmer 17 die Kolben bewegt (vgl. auch Fig. 2). Ferner ist der Wegsensor 11 der dargestellten Ausführungsform ein lineares Potentiometer. Alternativ kommen bei MRT-Einsatz bei gleicher Anordnung der Wegsensoren 11 gemäß Fig. 1 b auch optoelektronische Wegmessverfahren zum Einsatz.

Fig. 2 zeigt die Ausführungsform der Dosiervorrichtung 6 in perspektivischer Schnittdarstellung mit Wechselmagazin 7, die Einwegspritzen 5 mit Kolben 10 und dem Antrieb 9 aus mehreren Teilen. In der dargestellten Ausführungsform ist der Antrieb mit einem Elektromotor 15 ausgestattet, welcher über einen Spindeltrieb 16 mit Mitnehmer 17 die Kolben 10 bewegt.

Soll die Dosiervorrichtung 6 in einem MRT einsetzbar sein, sind zur Vermeidung von magnetischen Störungen für die Dosiervorrichtung 6 ausschließlich nicht magnetisierbare Komponenten einsetzbar. Als Antriebe bieten sich hierzu pneumatische oder hydraulische Turboantriebe, einseitig oder beidseitig wirkende hydraulische oder pneumatische Druckkolbenantriebe oder im eingeschränkten Fall auch Kardan-, Bowdenzug- oder Seilzugantriebe zu außerhalb des MRT liegenden Elektroantrieben sowie Piezoantriebe an.

Die Injektionsnadel 1 wird, wie in Fig. 1 a dargestellt, in einem reversibel zustellbaren und wieder lösbaren Nadelgreifer 13 eingespannt. Der Nadelgreifer 13 ist wiederum auf einer angetriebenen Verfahrvorrichtung 14 befestigt, welcher axial zur Injektionsnadel 1 verstellbar ist. Auf diese Weise ist die Injektionsnadel 1 mit dem Nadelgreifer 13 in einer bestimmten Weglänge verschiebbar. Soll die Injektionsnadel 1 weiter vorgeschoben werden, wird der Nadelgreifer 13 gelöst und mit der Verfahrvorrichtung 14 ohne Injektionsnadel 1 zurückgescho-ben, um die Injektionsnadel 1 an zurückgeschobener anderen Stelle wieder aufzunehmen und diese weiter vorzuschieben. Je nach Länge der Injektionsnadel 1 ist dieser Vorgang mehrfach wiederholbar.

Der besondere Vorteil dieser Injektionsnadelvorschubvorrichtung ist dessen kompakte Bauart unter Vermeidung größerer Kolben oder Linearantriebe. Somit sind die sehr beengten Platzverhältnisse in einem Kanal insbesondere eines MRT's ohne Einschränkung des Vorschubbereiches der Injektionsnadel 1 in vorteilhafter Weise nutzbar. Das System erlaubt die Platzierung aller Systemkomponenten in direkter Patientennähe, d.h. innerhalb eines CT's oder MRT's, um so das Totvolumen in sonst langen Medikamentenzuleitungen zu minimieren.

Ein weiterer Vorteil dieser Injektionsnadelvorschubvorrichtung liegt in der kurzen freien Nadellänge zwischen Nadelgreifer 13 und Einstichpunkt auch für längere Nadellängen, womit sich die Gefahr eines Ausknickens vor allem dünner biegsamer Injektionsnadeln während des Einstichs erheblich reduziert.

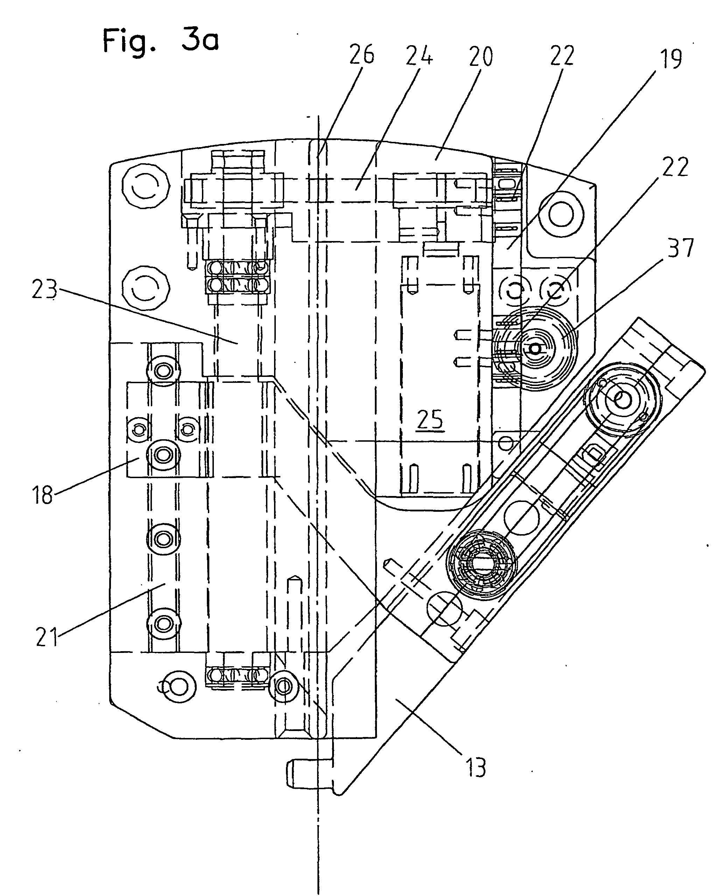

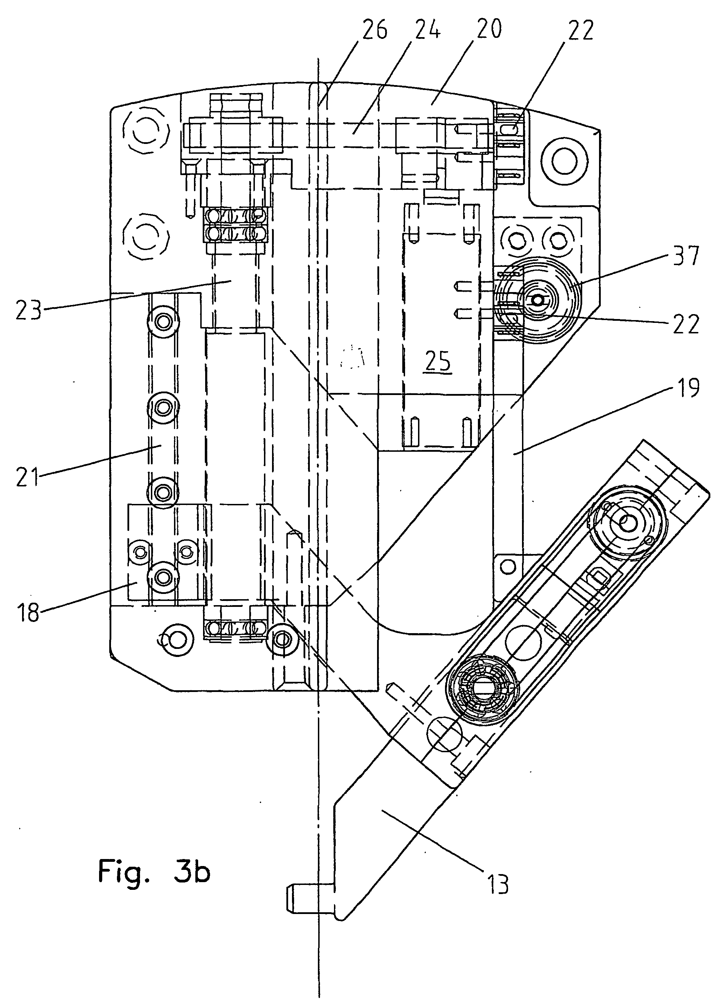

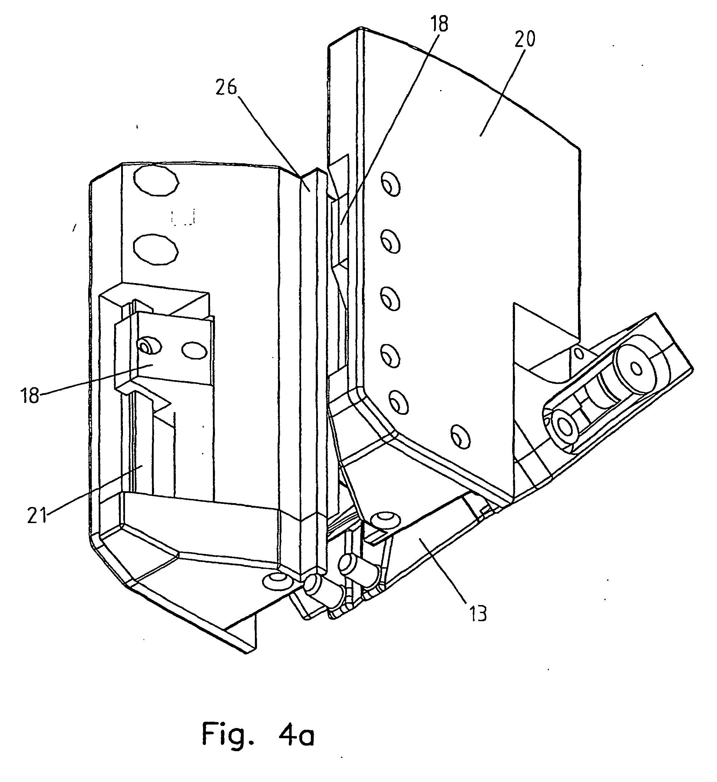

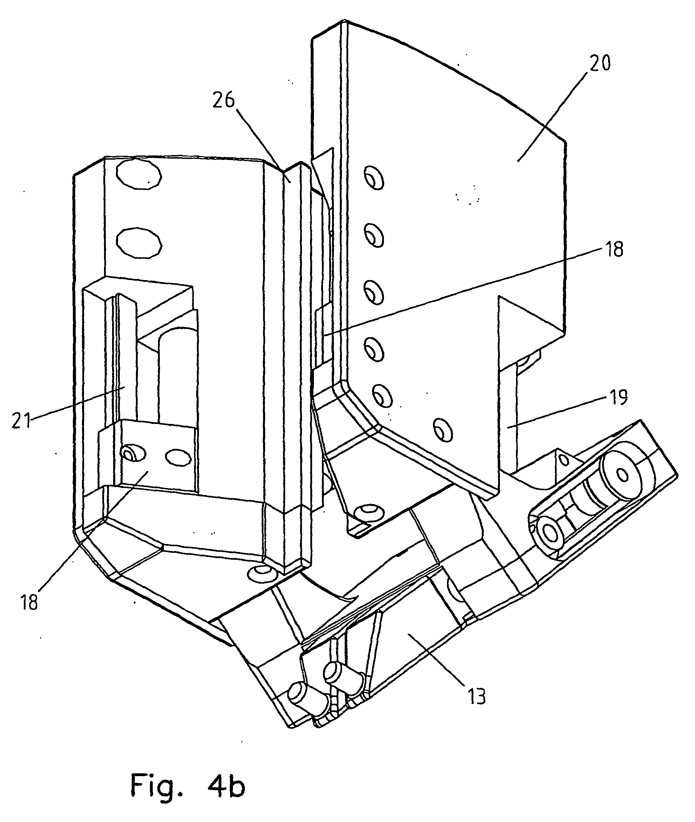

Fig. 3 a und b zeigen die Injektionsnadelvorschubvorrichtung ohne Injektionsnadel als Schnittdarstellung, Fig. 4a und b als perspektivische Darstellung, wobei die jeweils mit a bezeichneten Figuren den Nadelgreifer 13 in der zurückgeschobenen, die jeweils mit b bezeichneten Figuren den Nadelgreifer 13 in vorgeschobenen Position zeigen. Die Verfahrvorrichtung besteht dabei aus zwei Schlitten 18 und einer Zahnstange 19, welche mit dem Nadelgreifer 13 verbunden sind sowie auf zwei mit dem Gehäuse 20 der Injektionsnadelvorschubvorrichtung befestigten Schienen 21 sowie auf einer Führung 22 im Gehäuse 20 geführt sind. Die Verstellbewegung erfolgt im Ausführungsbeispiel über einen Spindelantrieb 23, welcher über einen Riemen 24 in der dargestellten Ausführungsform von einem Elektromotor 25 angetrieben wird.

Die Position des Nadelgreifers 13 wird in der dargestellten Form über ein Drehpotentiometer 37 mit Ritzel, welches über eine Zahnstange die Verschiebung des Nadelgreifers 13 in der Schiene 21 in der Führung 22 misst, bestimmt.
Die Injektionsnadel wird durch Führungsleisten 26 im Gehäuse 20 in axialer Richtung ausgerichtet und geführt, wobei die in Figuren 1 a und 1 b dargestellte Mischkammer 36 als Träger der Injektionsnadel 1 dient und die Außenkontur der Mischkammer 36 mit entsprechenden Führungsnuten 27 (siehe Fig. 1 a und 1 b) ausgestattet sein muss. Alternativ hierzu kann die Mischkammer auch auf einem separaten Träger, welches als separates Bauteil mit den erforderlichen Führungsnuten ausgestattet ist, befestigt sein. In diesem Fall ist die Mischkammer gemeinsam mit der Injektionsnadel und den Schläuchen von der Injektionsnadelvorschubvorrichtung lösbar. Das Gehäuse 20 ist im Bereich der Führungsleisten 26 offen gestaltet, sodass diese für ein Einsetzen der Injektionsnadel in die Injektionsnadelvorschub-vorrichtung sowie für die Rückschlagventile 3 und Schläuche 4 von oben, unten und von der Seite her zugänglich ist und darüber hinaus dem Arzt die visuelle Kontrolle des Einstichvorganges ermöglicht.

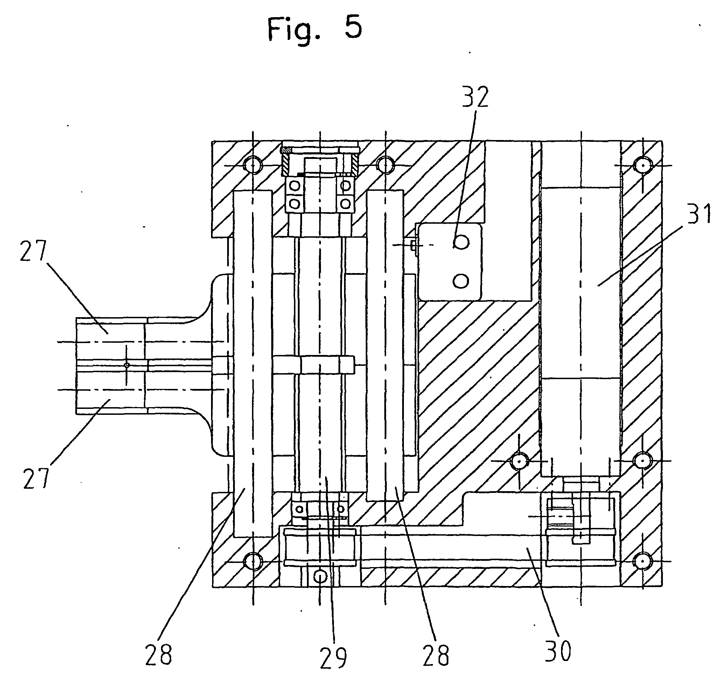

Einen Schnitt durch der Nadelgreifer 13 zeigt Fig. 5 in einer Schnittdarstellung. Diese besteht insbesondere aus den beiden Backen 27, welche auf zwei Führungsstiften 28 geführt werden und durch einen synchron gegenläufig wirkenden Spindelantrieb 29 gegeneinander bewegt werden. Der Spindelantrieb 29 wird über einen Riemen von einem Elektromotor 31 angetrieben, wobei mit einem Endschalter 32 zum Abschalten des Elektromotors 31 die Auseinanderbewegung der Backen 27 zueinander begrenzt wird.

Für einen Einsatz dieser Injektionsnadelvorschubvorrichtung am Patienten im Kanal eines MRT sind zur Vermeidung von magnetischen Störungen ausschließlich nicht magnetisierbare Komponenten einsetzbar. Somit müssen auch sämtliche Elektromotoren durch pneumatische oder hydraulische Turboantriebe, Piezoantriebe, einseitig oder beidseitig wirkende hydraulische oder pneumatische Druckkolbenantriebe oder im eingeschränkten Fall auch durch. Kardan-, Bowdenzug- oder Seilzugantriebe zu außerhalb des MRT liegenden Elektroantrieben ersetzt werden.

Für eine bessere Sterilisierbarkeit kann die Injektionsnadelvorschubvorrichtung als zweigeteilte Bauform ausgeführt werden, wobei die Injektionsnadel 1 und der Nadelgreifer 13 konstruktiv zur ersten, sterilisierbaren Baugruppe und die Vorfahrvorrichtung 14 zur zweiten, nicht sterilen Baugruppe gehören. Die erste Baugruppe ist nach jedem Eingriff von zweiten Baugruppe zu trennen und zu sterilisieren, während den zweiten Baugruppe mit einer weiteren sterilen ersten Baugruppe aufrüstbar und damit wieder einsetzbar ist.

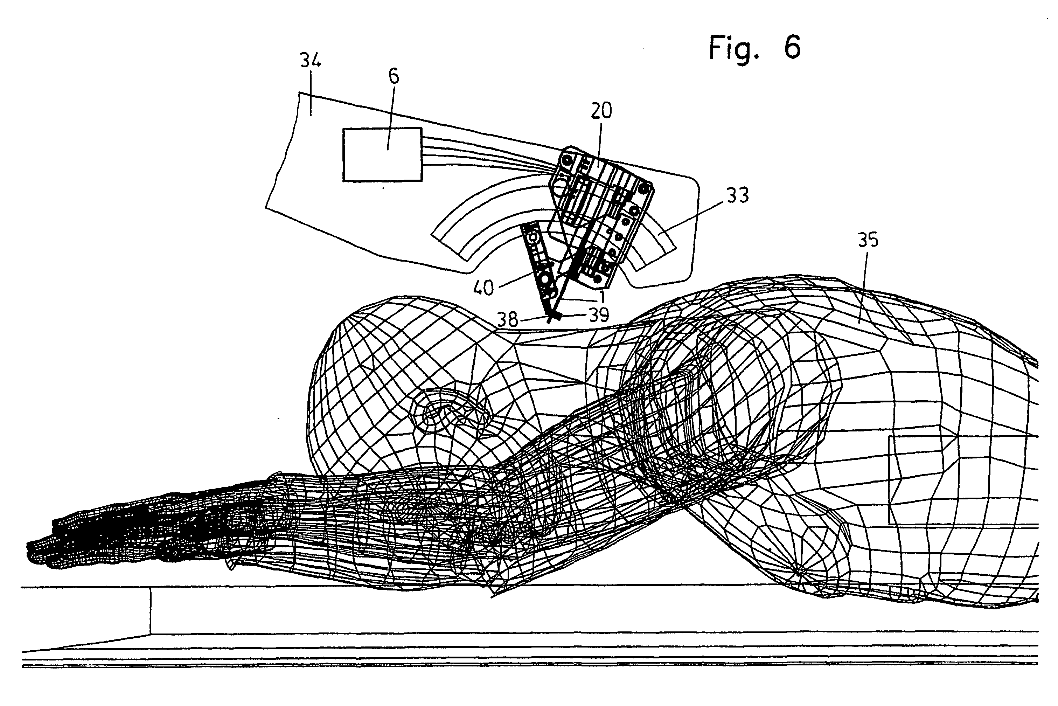

Für den beengten Einsatz der erfindungsgemäßen Vorrichtung im CT oder MRT wird das Gehäuse 20 der Injektionsnadelvorschubvorrichtung, wie in Fig. 6 dargestellt, auf einer C-Bogenführung 33 gemäß des Standes der Technik motorisch verfahrbar geführt und auf einem Träger 34 über den liegenden Patienten 35 manipulatorgesteuert positioniert. Die C-Bogenführung 33 sowie die auf einer nicht dargestellten manipulatorgesteuerten Schwenkvorrichtung für den Träger 34 ermöglichen dabei eine Schwenkung der Injektionsnadelvorschubvorrichtung um alle Raumfreiheitsgrade beispielsweise um einen fiktiven, ortsfesten Einstichpunkt 38, in den die Injektionsnadel 1 aus verschiedenen Richtungen einstechbar ist. Ferner ist die Dosiervorrichtung 6 auf dem Träger 34 nahe der Injektionsnadelvorschubvorrichtung angeordnet, womit die Länge der Schläuche erheblich reduzierbar ist. Eine kurze Länge der Schläuche bewirkt insbesondere eine Minimierung von Druckänderungen und Elastizitätseinflüssen der Schläuche und der Medikamente im Schlauch und dient damit insbesondere der genaueren Dosierung der Medikamente.

Alternativ ist die Aufgabe der beschriebenen C-Bogenführung 33, nämlich die Schwenkung der Injektionsnadelvorschubvorrichtung oder eines anderen medizinischen Instrumentes, auch mit einer angetriebenen Gelenkhalterung oder Drehscheibe, kombiniert mit einer rechnergestützten Manipulatorsteuerung realisierbar.

Ferner ist an den Träger 34 ein Sensor 39 befestigt, welcher über eine Haltevorrichtung 40 auf der Höhe des fiktiven Einstichpunktes 38 so ausgerichtet wird, dass das Messvolumen um diesen fiktiven Einstichpunkt 38 liegt. Beispielsweise kann dieser Sensor als ringförmiger Kontaktsensor ausgebildet sein, wobei der fiktive Einstichpunkt 38 innerhalb der Ringfläche liegt. Auf diese Weise lässt sich der fiktive Einstichpunkt 38 exakt auf einer Oberfläche, beispielsweise der Hautoberfläche des Patienten 35 anfahren. Der Patient 35 kann sich somit durch den Kontakt des Sensors 39 auf der Haut auf den bevorstehenden Einstich vorbereiten, womit sich die Gefahr eines Zuckens des Patienten beim Einstich erheblich reduziert. Der Sensor 39 überwacht somit die Bewegung des Patienten. Unterstützend oder alternativ hierzu kann auf dieser Oberfläche eine sensorisch erkennbare Markierung aufgetragen sein, welche mit nicht dargestellten optischen Detektoren erkennbar ist und manipulatorgesteuert mit dem fiktiven Einstichpunkt der Injektions-nadel in Übereinstimmung bringbar ist.
[1] Medrad Medizinische Systeme GmbH: Das Spectris MR-Injektionssystem, Broschüre 1997 der Medrad Inc., USA

### Bezugszeichenliste:

- 1: Infusionsnadel
- 2: Drucksensor
- 3: Rückschlagventil
- 4: Schlauch
- 5: Einwegspritze
- 6: Dosiervorrichtung
- 7: Wechselmagazin
- 8: Richtung
- 9: Antrieb
- 10: Kolben
- 11: Wegsensor
- 12: Endschalter
- 13: Nadelgreifer
- 14: Verfahrvorrichtung
- 15: Elektromotor
- 16: Spindeltrieb
- 17: Mitnehmer
- 18: Schlitten
- 19: Zahnstange
- 20: Gehäuse
- 21: Schiene
- 22: Führung
- 23: Spindelantrieb
- 24: Riemen
- 25: Elektromotor
- 26: Führungsleisten
- 27: Backen
- 28: Führungsstifte
- 29: Spindelantrieb
- 30: Riemen
- 31: Elektromotor
- 32: Endschalter
- 33: C-Bogenführung
- 34: Träger
- 35: Patient
- 36: Mischkammer
- 37: Drehpotentiometer
- 38: Fiktiver Einstichpunkt
- 39: Sensor
- 40: Haltevorrichtung

## Patentansprüche

1. Vorrichtung zur Injektion von medizinischen Präparaten in einen Patientenkörper während einer Untersuchung im Computertomographen, CT, oder im Magnetresonanztomographen, MRT, oder endoskopisch,
bestehend aus
a) einer Injektionsnadel (1) mit
b) einer separaten Dosiervorrichtung (6) für die Bereithaltung und sensorkontrollierten Förderung der medizinischen Präparate, wobei die Dosiervorrichtung (6) aufgrund ihrer geometrischen Abmessung sowie ihrer Material-, Sensor- und Antriebstechnik mit in den CT oder MRT einschiebbar und dort funktionsfähig einsetzbar ist sowie
c) einem Schlauch (4) für den Transport der medizinischen Präparate von der Dosiervorrichtung (6) in die Injektionsnadel,
**dadurch gekennzeichnet, dass**
d) die Injektionsnadel (1) in einer Injektionsnadelvorschubvorrichtung für ein Einstechen der Injektionsnadel in den Patienten (35) und gleichzeitiges Injizieren von Medikamenten untergebracht ist, wobei die Injektionsnadelvorschubvorrichtung für die Injektionsnadel (1) aus einer Nadelführung, einem Nadelgreifer (13) und einem Linearantrieb (14) für die parallel zur Injektionsnadel (1) gerichteten Verschiebung des Nadelgreifers (13) mit oder ohne Injektionsnadel besteht,
e) der Schlauch (4) mit einem Rückschlagventil (3) für den Einbahn-Transport der medizinischen Präparate ausgestattet ist sowie
f) zwischen Dosiervorrichtung (6) und Injektionsnadel (1) ein Drucksensor (2) für die Infusionsüberwachung vorgesehen ist.

2. Vorrichtung nach Anspruch 1, bei dem sämtliche Komponenten der Vorrichtung aus nichtmagnetischen Materialien bestehen, die Antriebe der Vorrichtung zudem kein magnetisches oder elektrisches Feld benötigen oder erzeugen, sondern pneumatische oder hydraulische Antrieb oder Piezo-Antriebe sind oder eine außerhalb des MRT erzeugte Antriebsleistung mechanisch über Kardan-, Bowdenzug-oder Seilantriebe oder hydraulisch oder pneumatisch über Druckkolbenantriebe zur Vorrichtung übertragbar sind.

3. Vorrichtung nach Anspruch 1 oder 2, bei dem die Injektionsnadel (1) über Schwenkvorrichtungen in allen Raumfreiheitsgraden um einen fiktiven Einstichpunkt (38) bewegbar ist.

4. Vorrichtung nach Anspruch 3, **dadurch gekennzeichnet, dass** an der Vorrichtung ein auf der Haut des Patienten aufliegender Sensor (39), der pneumatisch oder hydraulisch oder optoelektronisch arbeitet, um den und auf der Höhe des fiktiven Einstichpunkts (38) angeordnet ist.

5. Vorrichtung nach einem der Ansprüche 1 bis 4, **dadurch gekennzeichnet, dass** die Dosiervorrichtung (6) ein Wechselmagazin (7) zum unverwechselbaren Einlegen und Nachfüllen einer der Einwegspritzen (5) sowie einem Antrieb (9) mit Mitnehmer (17) zur Betätigung der Spritze enthält.

6. Vorrichtung nach Anspruch 5, **dadurch gekennzeichnet, dass** in das Wechselmagazin (7) mehrere Einwegspritzen (5) einlegbar sind und diese jeweils unabhängig voneinander durch einen eigenen Antrieb (9) mit Mitnehmer (17) in der Dosiervorrichtung (6) betätigbar sind, von jeder Spritze ein eigener Schlauch (4) zu einer Mischkammer (36) einer gemeinsamen Injektionsnadel (1) führt und diese Schläuche (4) kurz vor dem Anschluss an die Injektionsnadel je ein Rückschlagventil (3)aufweisen.

7. Vorrichtung nach einem der Ansprüche 1 bis 4, **dadurch gekennzeichnet, dass** die Injektionsnadelvorschubvorrichtung zweiteilig modular aufgebaut ist, wobei die Injektionsnadel (1) und der Nadelgreifer (13) zum ersten, sterilisierbaren Teil und die Verfahrvorrichtung (14) zum zweiten, nicht sterilen Teil gehören.

## Claims

1. Device for the injection of medical substances into a patient's body during an examination in a computer tomograph, CT, or in the magnetic resonance tomograph, MRT, or during an endoscopic examination, consisting of
a) an injection needle (1) with
b) a separate dosage unit (6) for the storage and sensor-controlled metering of the medical substances, with this dosage unit (6) having such geometrical dimensions and being equipped with such materials, sensors, and driving technology that it can also be pushed into the CT or MRT and works inside, as well as
c) a hose (4) for the transport of the medical substances from the dosage unit (6) into the injection needle,
**characterized by**
d) the injection needle (1) being accommodated in an injection needle feed unit for stabbing the injection needle into the patient (35) and the simultaneous injection of medicine, with the injection needle feed unit of the injection needle (1) consisting of a needle guide unit, a needle gripper (13), and a linear drive (14) for shifting the needle gripper (13) with or without the injection needle parallel to the latter (1),
e) the hose (4) being equipped with a check valve (3) for the one-way transport of the medical substances, and
f) a pressure sensor (2) being installed between the dosage unit (6) and the injection needle (1) for infusion monitoring.

2. Device according to claim 1, with all components of the device being made of non-magnetic materials and the drives of the device neither needing nor generating a magnetic or electric field, but representing pneumatic or hydraulic drives or piezodrives, or a driving force generated outside of the MRT being transmitted mechanically to the device using cardan, Bowden wire or rope drives or force transmission taking place hydraulically or pneumatically by pressure piston drives.

3. Device according to claim 1 or 2, with the injection needle (1) being movable around a fictitious insertion point (38) in all spatial degrees of freedom due to the use of sluing mechanisms.

4. Device according to claim 3, **characterized by** a pneumatic or hydraulic or optoelectronic sensor (39) lying on the skin of the patient and being located around and on the level of the fictitious insertion point (38).

5. Device according to one of claims 1 through 4, **characterized by** the dosage unit (6) containing an exchange magazine (7) for the correct insertion and re-filling of one of the disposable syringes (5) and a drive (9) with pins (17) for the actuation of the syringe.

6. Device according to claim 5, **characterized by** the fact that several disposable syringes (5) may be inserted into the magazine (7) and that these syringes may be actuated separately by an own drive (9) with pins (17) in the dosage unit (6), with each syringe being connected with the mixing chamber (36) of the joint injection needle (1) via a separate hose (4), and each of these hoses (4) being equipped with a check valve (3) that is located upstream of the connection of the injection needle.

7. Device according to one of claims 1 through 4, **characterized by** the injection needle feed unit being designed in a modular manner with two parts, with the injection needle (1) and the needle gripper (13) being accommodated in the first, sterilizable part and the feed unit (14) belonging to the second, non-sterile part.

## Revendications

1. Dispositif d'injection de préparations médicales dans le corps d'un patient pendant un examen au tomodensitomètre (CT) ou par tomographie à résonance magnétique (MRT) ou par voie endoscopique, consistant en
a) une aiguille d'injection (1 ) avec
b) un dispositif de dosage séparé (6) pour tenir les préparations médicales prêtes et pour les administrer sous contrôle par capteur, le dispositif de dosage (6), grâce à ses dimensions géométriques et sa technologie de matériau, sa technique sensorielle et d'entraînement pouvant être enfiché dans le CT ou le MRT et fonctionner dans ceux-ci, ainsi qu'en
c) un tuyau (4) pour le transport des préparations médicales du dispositif de dosage (6) vers l'aiguille d'injection
**caractérisé en ce que**
d) l'aiguille d'injection (1 ) est logée dans un dispositif pousseur d'aiguille pour piquer le patient (35) par l'aiguille et pour injecter en même temps des médicaments, le dispositif pousseur de l'aiguille d'injection (1 ) étant composé d'un guidage d'aiguille, d'un grappin d'aiguille (13) et d'un mécanisme de commande linéaire (14) pour le déplacement parallèle à l'aiguille d'injection (1) du grappin d'aiguille (13) avec ou sans aiguille d'injection,
e) le tuyau (4) est équipé d'un clapet antiretour (3) pour le transport en sens unique des préparations médicales et que
f) un capteur de pression est prévu entre le dispositif de dosage (6) et l'aiguille d'injection pour contrôler la perfusion.

2. Dispositif selon la revendication 1, où tous les composants du dispositif sont en matériaux non magnétiques, où en outre les mécanismes de commande du dispositif n'ont pas besoin de champ magnétique ou électrique et ne le produisent pas, mais sont des mécanismes de commande pneumatiques ou hydrauliques ou des piézo-commandes, ou bien qu'une puissance motrice produite à l'extérieur du MRT peut être transmise au dispositif mécaniquement par des Cardans, des câbles Bowden ou des commandes par câbles, ou hydrauliquement ou pneumatiquement par des commandes à piston de compression.

3. Dispositif selon l'une quelconque des revendications 1 ou 2, dans lequel l'aiguille d'injection (1) est orientable dans tous les degrés de liberté spatiaux autour d'un point d'injection fictif (38), grâce à des dispositifs de pivotement.

4. Dispositif selon la revendication 3, **caractérisé en ce qu'**un capteur (39) fonctionnant en mode pneumatique ou hydraulique ou optoélectronique, appliqué sur la peau du patient, est disposé autour et au niveau du point d'injection fictif (38).

5. Dispositif selon l'une quelconque des revendications 1 à 4, **caractérisé en ce que** le dispositif de dosage (6) contient un magasin interchangeable (7) pour l'introduction et le rechargement indubitable d'une des seringues jetables (5) ainsi qu'un mécanisme de commande (9) avec entraîneur (17) pour appliquer la seringue.

6. Dispositif selon la revendication 5, **caractérisé en ce que** plusieurs seringues jetables (5) peuvent être introduites dans le magasin interchangeable (7) et que celles-ci peuvent être appliquées indépendamment l'une de l'autre moyennant un mécanisme de commande propre (9) avec entraîneur (17) dans le dispositif de dosage, qu'un tuyau propre (4) conduit de chaque seringue vers une chambre de mélange (36) d'une aiguille d'injection commune (1 ) et que ces tuyaux (4) présentent chacun un clapet antiretour (3) immédiatement devant le raccord à l'aiguille d'injection.

7. Dispositif selon l'une quelconque des revendications 1 à 4, **caractérisé en ce que** le dispositif pousseur de l'aiguille d'injection posséde une structure modulaire en deux parties, l'aiguille d'injection (1) et le grappin d'aiguille (13) appartenant à la première partie, stérilisable, et le dispositif de déplacement (14) appartenant à la deuxième partie, non stérile.
